# EUROPÄISCHE PATENTSCHRIFT

(11) **EP 0 830 122 B1**
(45) Veröffentlichungstag und Bekanntmachung des Hinweises auf die Patenterteilung: **02.11.2000**
(21) Anmeldenummer: 96919543.7
(22) Anmeldetag: 15.05.1996
(51) Int. Cl.: A61F 13/15, A61F 13/68

(54) **HYGIENEHOSE MIT EINEM HOSENELEMENT UND EINEM SAUGPOLSTER**
SANITARY PANTS WITH PANTS COMPONENT AND ABSORBENT PAD
CULOTTE HYGIENIQUE COMPRENANT UN ELEMENT DE CULOTTE ET UN COUSSIN ABSORBANT

(30) Priorität: 16.05.1995 DE 29508007 U
(43) Veröffentlichungstag der Anmeldung: 25.03.1998
(73) Patentinhaber: Riehl, Rita, 92348 Berg (DE)
(72) Erfinder: Riehl, Rita, 92348 Berg (DE)
(74) Vertreter: Hafner, Dieter, Dr.rer.nat., Dipl.-Phys.
(86) Internationale Anmeldenummer: DE9600854
(87) Internationale Veröffentlichungsnummer: WO9636305

(56) Entgegenhaltungen:
- EP-A- 0 430 443
- DE-U- 9 113 758
- DE-U- 9 211 983
- DE-U- 9 314 628
- US-A- 4 955 880
- US-A- 5 217 447

## Beschreibung

Die Erfindung betrifft eine Hygienehose mit einem Hosenelement und einem Saugpolster mit den weiteren Merkmalen des Oberbegriffs des Patentanspruchs.

Als Stand der Technik sind textile Hygienehosen bekannt, die für Säuglinge, Kleinkinder, inkontinente Personen jeden Alters sowie beispielsweise für Frauen zur Monatshygiene mit oder ohne herausnehmbares Saugpolster ausgebildet sind. Derartige Hygienehosen sind zum einen als Einweghosen bekannt, was allerdings sowohl in wirtschaftlicher als auch in ökologischer Hinsicht als nachteilig anzusehen sind.

Desweiteren sind als Alternative zu den Einwegprodukten mehrfach verwendete Hygienehosen bekannt. Hier unterscheidet man Modelle mit fest eingebrachten oder eingenähten Saugpolstern, welche den Nachteil aufweisen, daß die gesamte Hose samt Saugpolster gewaschen und getrocknet werden muß, was insbesondere in kühleren Jahreszeiten oder im Winter mit nicht unerheblichen Trockenzeiten verbunden ist. Außerdem ist eine vollständige Austrocknung und Durchtrocknung einer derartigen Mehrweghose nicht sicher gewährleistet, so daß es auf Dauer zum Auftreten von Stockflecken, Schimmel- oder Pilzbildung kommen kann und damit hygienisch unerwünschte Begleitwirkungen auftreten.

Andere Mehrfachhosen besitzen von der Außenhose lösbare Saugelemente, welche einzeln gewaschen werden können, jedoch aufgrund ihrer kompakten Form eine verhältnismäßig große Dicke besitzen, so daß sich wiederum das Problem der vollständigen Durchtrocknung und der langfristigen hygienischen Verträglichkeit stellt.

Die bisher üblichen Hygienehosen sind zudem nur in wenigen standardisierten Ausführungen erhältlich, welche in ihrer Schnittführung nicht an die Körperform des Trägers individuell oder annähernd angepaßt ist. Die verwendeten Mehrfachhygienehosen besitzen demnach häufig einen geringen Tragekomfort.

Aus der EP 0 430 443 geht ein Hygieneartikel hervor, der mindestens ein stark absorbierendes Saugpolster aufweist, welches lösbar mit dem Hygieneartikel verbunden ist.

In der US 4 995 880 wird ein mit Ausnehmungen versehenes Saugpolster beschrieben, welches in eine Hygienehose zur Fixierung über hindurchgreifende Befestigungselemente einbringbar ist. Die dort beschriebene Hygienehose benötigt konkrete vorfabrizierte Saugpolster

Der Erfindung liegt die Aufgabe zugrunde, eine Hygienehose anzubieten, welche eine Fixierung unterschiedlich geschnittener und gefalteter Saugpolster ermöglicht.

Die Aufgabe wird durch den kennzeichnenden Teil des Patentanspruchs 1 gelöst. Vorteilhafte Ausführungsformen der Erfindung werden durch die Unteransprüche 2 - 14 realisiert.

Die vorgeschlagene Hygienehose weist ein Hosenelement auf, welches aus einem Mittelabschnitt, zwei identischen seitlichen Beinabschnitten und zwei Überdeckungsbereichen besteht, wobei alle diese Hosenelemente durch Vernähung, Verklebung, Verschweißung oder eine sonstige Technologie miteinander verbunden sind. Damit stellt die vorgeschlagene Hygienehose eine Mehrschnittkonstruktion dar, die durch Einzelanpassung des Mittelabschnitts, der Beinabschnitte bzw. der Überdeckungsbereiche individuell an die anatomischen Gegebenheiten von Säuglingen, Kleinkindern oder Erwachsenen angepaßt werden kann.

Aufgrund des "modularen" Aufbaus kann die Gesamtschnittform der vorgeschlagenen Hygienehose durch Einzelabänderungen individuell und einfach festgelegt werden. Zwischen den Überdeckungsbereichen und dem Mittelabschnitt befinden sich Vertiefungen, in welche ein herausnehmbares Saugpolster eingesteckt werden kann. Im eingesteckten Zustand trägt das Saugpolster zu einer Stabilisierung des Hosenelementes der Hygienehose bei.

In die vorgeschlagene Hygienehose können Saugpolster unterschiedlicher Abmessungen eingesteckt werden, welche zum Schutz vor Verrutschen über Befestigungselemente gesichert werden können. Über diese Befestigungselemente kann eine lösbare Verbindung zwischen den Überdeckungsbereichen und dem Mittelabschnitt sowie gegebenenfalls den Beinabschnitten der Hygienehose hergestellt werden, wodurch das zwischen Überdekkungsbereich und Mittelabschnitt bzw. Beinabschnitten eingesteckte Saugpolster verrutschsicher aufgenommen wird.

Vorteilhafterweise werde als Befestigungselemente Klettverschlüsse, Häkchenverschlüsse oder Druckknopfverschlüsse verwendet, wobei an einer einzigen Hygienehose auch abschnittsweise unterschiedliche Verschlußprinzipien angewendet werden können. So kann in gering beanspruchten und häufig zu lösenden Bereichen beispielsweise ein Klettverschluß verwendet werden, während an anderen Stellen ein strapazierfähiger und belastbarer Häkchenverschluß Anwendung finden kann.

Vorteilhafterweise sind die jeweiligen Befestigungselemente zweiteilig ausgebildet und zum einen am Überdeckungsbereich und zum anderen an entsprechender gegenüberliegender Stelle an dem Mittelabschnitt bzw. den Beinabschnitten befestigt. Bei einer separaten Herstellung der einzelnen Stoffbereiche der vorgeschlagenen Hygienehose können die jeweiligen Befestigungselemente direkt eingearbeitet werden.

Vorteilhafterweise ist das einsteckbare Saugpolster aufklappbar gestaltet und wird mehrfach gefaltet in das Hosenelement der Hygienehose eingesteckt. Beim Waschen wird das Saugpolster aus den Vertiefungen des Hosenelementes herausgezogen, aufgeklappt und kann somit aufgrund seiner geringen Gesamtdicke vorteilhaft gewaschen und vollständig durchtrocknet werden Zur besseren Anpassung des Saugpolsters an die anatomische Form des Trägers wird vorteilhafterweise im Rückenbereich des Trägers eine längere Schnittführung, verwendet.

Durch Aufbringen einer feuchtigkeitshemmenden Vliesauflage wird die feuchtigkeitsresorbierende und abschirmende Wirkung des Saugpolsters zusätzlich noch verstärkt.

Zur weiteren Verbesserung des Tragekomforts sind an verschiedenen Elementen der Hygienehose - z.B. an den Beinabschnitten, in den Überdeckungsbereichen oder im Mittelabschnitt - elastische Elemente aufgearbeitet, welche die Form der Hygienehose im getragenen Zustand vollständig an die Anatomie des Trägers anpassen. Als derartige elastische Elemente können beispielsweise Gummilitzen verwendet werden.

Zum Verschluß der vorgeschlagenen Hygienehose besitzt das zugehörige Hosenelement vorteilhafterweise mindestens zwei miteinander korrespondierende Verschlußelemente, die beispielsweise als Klettverschlüsse ausgebildet sein können.

Die geschilderte Mehrschnittkonstruktion der vorgeschlagenen Hygienehose erlaubt es, einzelne Elemente - z.B. nur den Mittelabschnitt oder nur die Beinabschnitte aus Sondermaterialien, z.B. besonders Feuchtigkeitsdichtematerial oder atmungsaktiven Kunststoffmaterial - auszubilden und damit bestimmte an die Hygienehose gestellte Anforderungen besonders gut zu erfüllen.

Allgemein kann es von Vorteil sein, die gesamte Hygienehose - also das gesamte Hosenelement sowie das Saugpolster - aus kochfestem Material auszubilden Ein besonders natürliches Tragegefühl stellt sich bei der Verwendung von natürlichen Textilien, wie z.B. Baumwolle oder Viskose ein.

Die Erfindung ist anhand von Ausführungsbeispielen in den Zeichnungsfiguren näher erläutert Es zeigen:
- Fig. 1: eine Gesamtansicht der vorgeschlagenen Hygienehose ohne Saugpolster im aufgeschlagenem Zustand,
- Fig. 2: eine Gesamtansicht des Saugpolsters im aufgeschlagenen Zustand,
- Fig. 3: eine Gesamtansicht der vorgeschlagenen Hygienehose mit eingestecktem Saugpolsters im aufgeschlagenem Zustand sowie
- Fig. 4: eine Schnittansicht A-A nach Fig. 3.

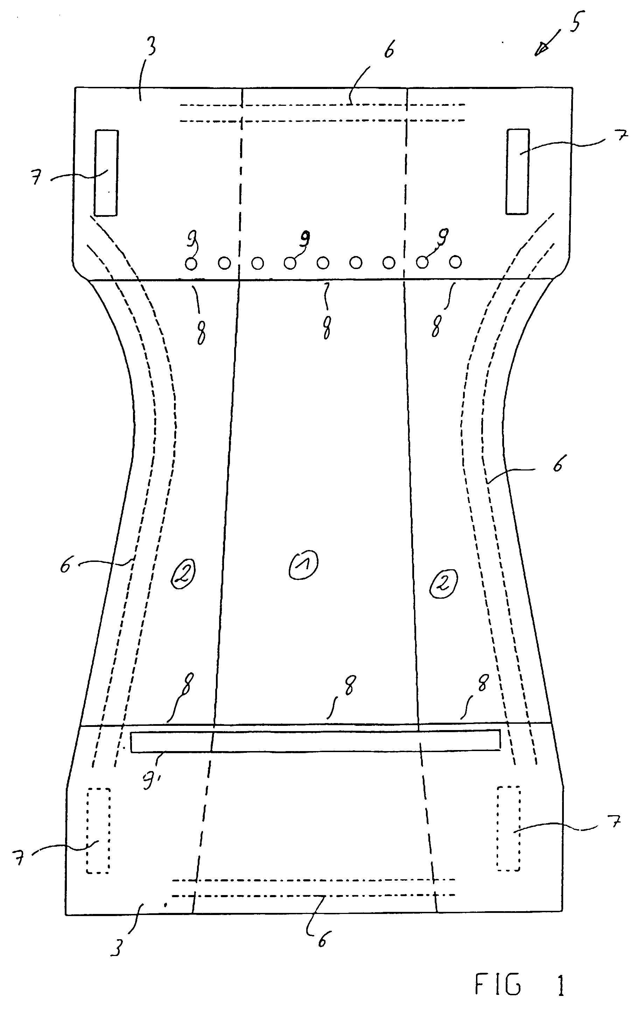

Zunächst wird auf Zeichnungsfigur 1 Bezug genommen. Das abgebildete Hosenelement 5 weist einen zentralen Mittelabschnitt 1 auf, an welchen sich links und rechts (identische) Beinabschnitte 2 anschließen. Auf die beispielsweise durch Vernähung miteinander verbundenen beiden Beinabschnitte 2 sowie den Mittelabschnitt 1 werden nun im oberen und unteren Endbereich (beispielsweise ebenfalls durch Vernähung) Überdekkungsbereiche 3 aufgebracht. Hierdurch entstehen zwischen den Überdeckungsbereichen 3 und den beiden Beinabschnitten 2 dem Mittelbereich 1 Vertiefungen 8, in welche beispielsweise das Saugpolster 4 (in Fig. 1 nicht abgebildet) eingesteckt werden kann.

Zur besseren anatomischen Anpassung an den Körper des Trägers ist das Hosenelement 5 mit elastischen Elementen 6 in den Überdeckungsbereichen 3 sowie in den Beinabschnitten 2 versehen.

Zum Verschluß des oberen und des unteren Endes des Hosenelementes 5 sind jeweils im oberen und unteren Ende korrespondierende Verschlußelemente 7 (beispielsweise Klettverschlüsse) angebracht.

Im Bereich der Vertiefungen 8 sind an den Überdeckungsbereichen 3 sowie an den Beinabschnitten 2 und dem Mittelabschnitt 1 korrespondierende Befestigungselemente 9 bzw 9' angebracht, über welche eine losbare Verbindung zwischen Überdeckungsbereich 3 sowie den Beinabschnitten 2 und dem Mittelabschnitt 1 hergestellt werden kann.

In Fig. 1 sind die Befestigungselemente 9 als Druckknöpfe und das Befestigungselement 9' als Klettverschluß ausgebildet.

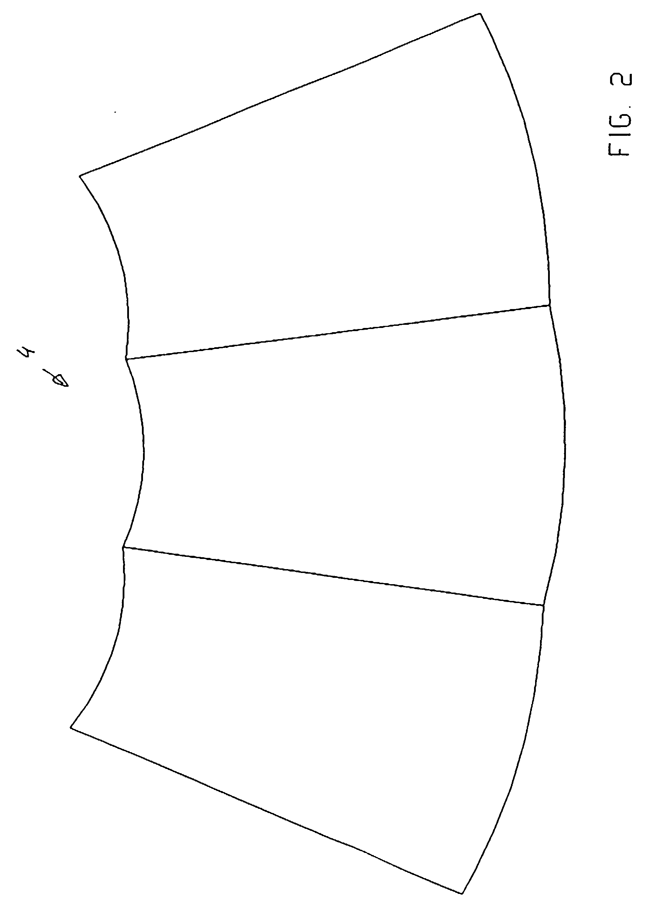

Fig. 2 zeigt das Saugpolster 4 in Einzeldarstellung und im aufgeklappten Zustand. Es ist ersichtlich, daß das Saugpolster 4 im unteren Bereich eine längere Schnittführung aufweist, was auch der Anpassung an die anatomischen Gegebenheiten im Rückenbereich des Trägers dient.

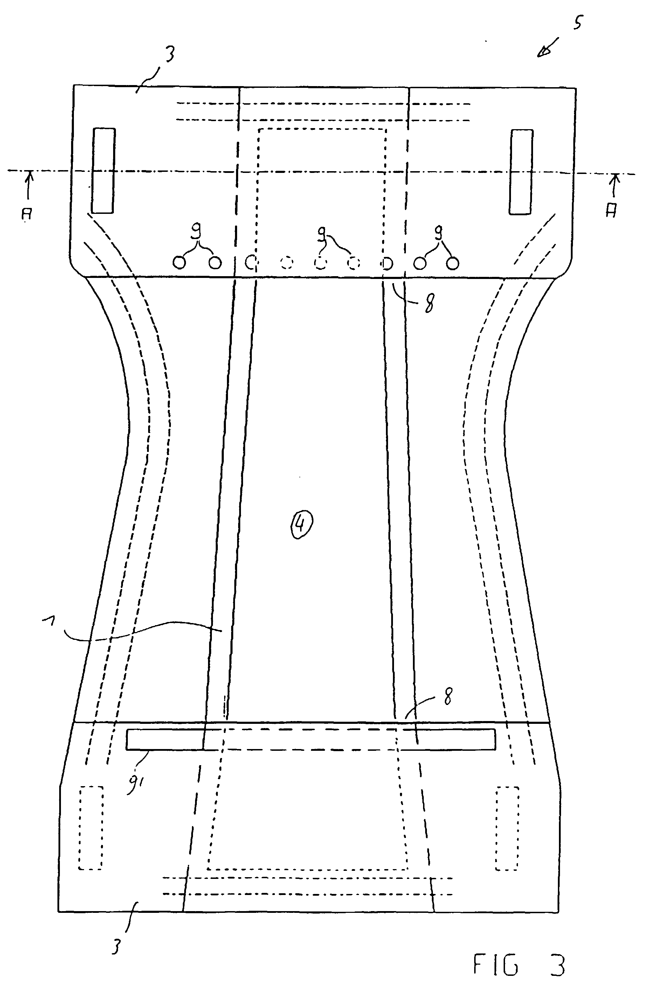

Fig. 3 zeigt das Hosenelement 5 mit in die Vertiefungen 8 zwischen Überdeckungsbereich 3 und Mittelabschnitt 1 eingestecktem Saugpolster 4. Beim Waschen der Hygienehose wird das Saugpolster 4 einfach aus den Vertiefungen 8 herausgezogen, aufgefaltet und diesem Zustand gewaschen und getrocknet.

Das eingesteckte Saugpolster 4 wird durch die Befestigungselemente 9 und 9' vor Verrutschungen gesichert. In Fig. 3 sind die durchgängig gezeichneten Befestigungselemente 9 geschlossen, und zwar bis an den Randbereich des Saugpolsters 4 heran, während die gestrichelt gezeichneten Befestigungselemente 9 geöffnet sind, um ein Einstecken des Saugpolsters 4 zu gewährleisten.

Das als Klettverschluß ausgebildete Befestigungselement 9' ist von beiden Seiten bis an die Ränder des Saugpolsters 4 geschlossen und im Überdeckungsbereich 3 des Saugpolsters 4 geöffnet. Insgesamt wird somit durch die teilweise geschlossenen und teilweise geöffneten Befestigungselemente 9 bzw 9' eine an die individuellen Abmessungen des jeweiligen eingelegten Saugpolsters 4 anpaßbare Seitenführung des Saugpolsters 4 erreicht.

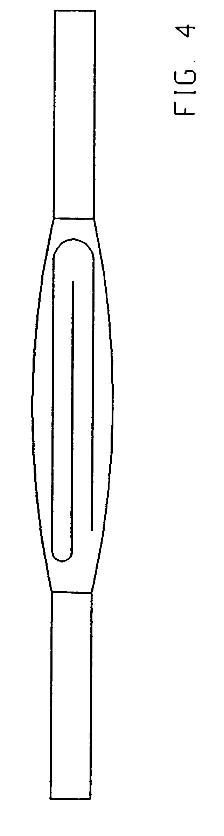

Fig 4 zeigt einen Schnitt A-A nach Fig 3 und laßt deutlich das zweifach gefaltete Saugpolster 4 erkennen, welche sind die Vertiefung 8 zwischen Überdeckungsbereich 3 und Mittelabschnitt 1 einefuhrt ist und don durch Reibschluß gehaltert wird Das derartige eingesteckte Saugpolster 4 tragt auch zu einer Verstärkung des Hosenelementes 5 im oberen und unteren Endbereich bei.

## Patentansprüche

1. Hygienehose, bestehend aus einem Hosenelement und einem Saugpolster, mit einem Mittelabschnitt, zwei seitlichen Beinabschnitten und zwei Überdeckungsbereichen, wobei das Saugpolster zur Herstellung einer lösbaren Verbindung mit dem Hosenelement in Vertiefungen zwischen die Überdeckungsbereiche und dem Mittelabschnitt einschiebbar ist und zur seitlichen Fixierung von Saugpolstern unterschiedlicher Abmessungen Befestigungselemente vorgesehen sind, die eine lösbare Verbindung zwischen den Überdeckungsbereichen einerseits und dem Mittelabschnitt und/oder den Beinabschnitten andererseits ermöglichen,
**dadurch gekennzeichnet, daß**
die das eingeschobene Saugpolster (4) überdeckenden Befestigungselemente (9) geöffnet und die sich an beiden Seiten des Saugpolsters (4) anschließenden Befestigungselementen (9) zur Fixierung des Saugpolsters (4) geschlossen und miteinander verbunden sind.

2. Hygienehose nach Anspruch 1,
**dadurch gekennzeichnet, daß**
die Befestigungselemente (9) aus zwei lösbar miteinander verbindbaren Teilen bestehen, wobei ein Teil am Überdeckungsbereich (3) und das korrespondierende zweite Teil am Beinabschnitt (2) oder am Mittelabschnitt (1) befestigt sind.

3. Hygienehose nach einem der vorhergehenden Ansprüche,
**dadurch gekennzeichnet, daß**
die Befestigungselemente (9) als Klettverschluß und/oder Häkchenverschluß und/oder Druckknopfverschluß ausgebildet sind.

4. Hygienehose nach einem der vorhergehenden Ansprüche,
**dadurch gekennzeichnet, daß**
der Mittelabschnitt (1), die beiden Beinabschnitte (2) und die beiden Überdekkungsbereiche (3) zur Bildung des Hosenelementes (5) miteinander vernäht, verklebt oder verschweißt sind.

5. Hygienehose nach einem der vorhergehenden Ansprüche,
**dadurch gekennzeichnet, daß**
die Beinabschnitte (2) und/oder die Überdeckungsbereiche (3) und/oder der Mittelabschnitt (1) elastische Elemente (6), insbesondere Gummilitzen aufweisen.

6. Hygienehose nach einem der vorhergehenden Ansprüche
**dadurch gekennzeichnet, daß**
das Hosenelement (5) mindestens zwei miteinander korrespondierende Verschlußelemente (7) zur Öffnung und zum Verschluß des Hosenelementes (5) aufweist.

7. Hygienehose nach Anspruch 6,
**dadurch gekennzeichnet, daß**
als Verschlußelemente (7) Klettverschlüsse verwendet werden.

8. Hygienehose nach einem der vorhergehenden Ansprüche,
**dadurch gekennzeichnet, daß**
die Paßform des Hosenelementes (5) durch einzelne und individuelle Anpassung des Mittelabschnitts (1) und/oder der beiden Beinabschnitte (2) sowie der beiden Überdeckungsbereiche (3) vorgenommen wird.

9. Hygienehose nach einem der vorhergehenden Ansprüche,
**dadurch gekennzeichnet, daß**
das Hosenelement (5) und/oder das Saugpolster (4) aus natürlichen textilem Material wie Bauwolle oder Viskose besteht.

10. Hygienehose nach einem der vorhergehenden Ansprüche,
**dadurch gekennzeichnet, daß**
das Hosenelement (5) und/oder das Saugpolster (4) aus kochfestem Material bestehen.

11. Hygienehose nach einem der vorhergehenden Ansprüche,
**dadurch gekennzeichnet, daß**
das Hosenelement (5) zumindest teilweise aus feuchtigkeitsdichtem Material ausgebildet ist.

12. Hygienehose nach einem der vorhergehenden Ansprüche,
**dadurch gekennzeichnet, daß**
das Hosenelement (5) zumindest teilweise aus atmungsaktivem Kunststoffmaterial ausgebildet ist.

13. Hygienehose nach einem der vorhergehenden Ansprüche,
**dadurch gekennzeichnet, daß**
das Saugpolster (4) anatomisch geformt ist und im Rückenbereich des Trägers eine längere Schnittführung aufweist.

14. Hygienehose nach einem der vorhergehenden Ansprüche,
**dadurch gekennzeichnet, daß**
das Hosenelement (5) durch Einstecken des Saugpolsters (4) in die Vertiefungen (8) zwischen Mittelabschnitt (1) und Überdeckungsbereichen (3) verstärkt wird.

## Claims

1. Sanitary pants, comprising a pants element and an absorbent pad, with a central portion, two lateral leg portions and two covering regions, the absorbent pad being adapted such that it can be pushed into depressions between the covering regions and the central portion to establish a releasable connection with the pants element, and fastening elements which permit a releasable connection between the covering regions on the one hand and the central portion and/or the leg portions on the other hand being provided for the lateral fixing of absorbent pads of different dimensions, characterized in that the fastening elements (9) covering the pushed-in absorbent pad (4) are open and the fastening elements (9) adjoining on both sides of the absorbent pad (4) for fixing the absorbent pad (4) are closed and connected to one another.

2. Sanitary pants according to Claim 1, characterized in that the fastening elements (9) comprise two parts which can be releasably connected to each other, one part being fastened to the covering region (3) and the corresponding second part being fastened to the leg portion (2) or to the central portion (1).

3. Sanitary pants according to one of the preceding claims, characterized in that the fastening elements (9) are formed as a Velcro-type closure and/or hook closure and/or stud closure.

4. Sanitary pants according to one of the preceding claims, characterized in that the central portion (1), the two leg portions (2) and the two covering regions (3) are sewn, adhesively bonded or fused to one another to form the pants element (5).

5. Sanitary pants according to one of the preceding claims, characterized in that the leg portions (2) and/or the covering regions (3) and/or the central portion (1) have elastic elements (6), in particular elastic braids.

6. Sanitary pants according to one of the preceding claims, characterized in that the pants element (5) has at least two closure elements (7) corresponding to each other for the opening and closing of the pants element (5).

7. Sanitary pants according to Claim 6, characterized in that Velcro-type closures are used as the closure elements (7).

8. Sanitary pants according to one of the preceding claims, characterized in that the pants element (5) is made to fit by single and individual adaptation of the central portion (1) and/or of the two leg portions (2) as well as of the two covering regions (3).

9. Sanitary pants according to one of the preceding claims, characterized in that the pants element (5) and/or the absorbent pad (4) consists of natural textile material such as cotton or viscose.

10. Sanitary pants according to one of the preceding claims, characterized in that the pants element (5) and/or the absorbent pad (4) consist of boil-resistant material.

11. Sanitary pants according to one of the preceding claims, characterized in that the pants element (5) is formed at least partially from moistureproof material.

12. Sanitary pants according to one of the preceding claims, characterized in that the pants element (5) is formed at least partially from breathable synthetic material.

13. Sanitary pants according to one of the preceding claims, characterized in that the absorbent pad (4) is anatomically shaped and is cut longer in the back region of the wearer.

14. Sanitary pants according to one of the preceding claims, characterized in that the pants element (5) is reinforced by inserting the absorbent pad (4) into the depressions (8) between the central portion (1) and the covering regions (3).

## Revendications

1. Culotte hygiénique comprenant un élément de culotte et un coussin absorbant, comportant une zone centrale, deux zones crurales latérales et deux régions de recouvrement, le coussin absorbant pouvant être inséré dans des renfoncements entre les régions de recouvrement et la zone centrale, en vue d'établir une liaison libérable avec l'élément de culotte, et des éléments de fixation, prévus pour le verrouillage latéral de coussins absorbants de dimensions différentes, autorisant une liaison libérable entre les régions de recouvrement, d'une part, et la zone centrale et/ou les zones crurales, d'autre part,
caractérisée par le fait que
les éléments de fixation (9) recouvrant le coussin absorbant (4) inséré sont ouverts, et les éléments de fixation (9) se rattachant aux deux côtés du coussin absorbant (4) sont fermés afin de verrouiller ledit coussin absorbant (4), puis reliés mutuellement.

2. Culotte hygiénique selon la revendication 1,
caractérisée par le fait que
les éléments de fixation (9) sont constitués de deux parties pouvant être reliées amoviblement l'une à l'autre, une partie étant fixée à la région de recouvrement (3), et la seconde partie correspondante étant reliée à la zone crurale (2) ou à la zone centrale (1).

3. Culotte hygiénique selon l'une des revendications précédentes,
caractérisée par le fait que
les éléments de fixation (9) sont réalisés sous la forme d'une fermeture auto-agrippante et/ou d'une fermeture à crochets et/ou d'une fermeture à bouton-pression.

4. Culotte hygiénique selon l'une des revendications précédentes,
caractérisée par le fait que
la zone centrale (1), les deux zones crurales (2) et les deux régions de recouvrement (3) sont solidarisées par couture, par collage ou par soudage, en vue de former l'élément (5) de culotte.

5. Culotte hygiénique selon l'une des revendications précédentes,
caractérisée par le fait que
les zones crurales (2) et/ou les régions de recouvrement (3) et/ou la zone centrale (1) présentent des éléments élastiques (6), notamment des cordons en caoutchouc.

6. Culotte hygiénique selon l'une des revendications précédentes,
caractérisée par le fait que
l'élément (5) de culotte présente au moins deux éléments de fermeture (7) se correspondant mutuellement, en vue de l'ouverture et de la fermeture dudit élément (5) de culotte.

7. Culotte hygiénique selon la revendication 6,
caractérisée par le fait que
des fermetures auto-agrippantes sont utilisées en tant qu'éléments de fermeture (7).

8. Culotte hygiénique selon l'une des revendications précédentes,
caractérisée par le fait que
le gabarit de l'élément (5) de culotte résulte d'une adaptation autonome et individuelle de la zone centrale (1) et/ou des deux zones crurales (2), ainsi que des deux régions de recouvrement (3).

9. Culotte hygiénique selon l'une des revendications précédentes,
caractérisée par le fait que
l'élément (5) de culotte et/ou le coussin absorbant (4) consiste(nt) en un matériau textile naturel, tel que du coton ou de la viscose.

10. Culotte hygiénique selon l'une des revendications précédentes,
caractérisée par le fait que
l'élément (5) de culotte et/ou le coussin absorbant (4) consiste(nt) en un matériau résistant à l'ébullition.

11. Culotte hygiénique selon l'une des revendications précédentes,
caractérisée par le fait que
l'élément (5) de culotte est réalisé, au moins en partie, en un matériau étanche à l'humidité.

12. Culotte hygiénique selon l'une des revendications précédentes,
caractérisée par le fait que
l'élément (5) de culotte est réalisé, au moins en partie, en une matière synthétique activant la respiration cutanée.

13. Culotte hygiénique selon l'une des revendications précédentes,
caractérisée par le fait que
le coussin absorbant (4) présente une forme anatomique, et comporte une coupe plus longue dans la zone dorsale de l'utilisateur.

14. Culotte hygiénique selon l'une des revendications précédentes,
caractérisée par le fait que
l'élément (5) de culotte est renforcé par emboîtement du coussin absorbant (4) dans les renfoncements (8) entre la zone centrale (1) et les régions de recouvrement (3).
